# EUROPÄISCHE PATENTANMELDUNG

(11) **EP 4 506 708 A1**
(43) Veröffentlichungstag der Anmeldung: **12.02.2025**
(21) Anmeldenummer: 23190884.9
(22) Anmeldetag: 10.08.2023
(51) Int. Cl.: G01R 33/36

(54) **MAGNETRESONANZVORRICHTUNG MIT SCHALTEINHEIT UND VERFAHREN ZUM BETRIEB EINER SOLCHEN**

(71) Anmelder: Siemens Healthineers AG, 91301 Forchheim (DE)
(72) Erfinder: Kimmlingen, Ralph, 90513 Zirndorf (DE)
(74) Vertreter: Siemens Healthineers Patent Attorneys

(57) **Zusammenfassung**

Die Erfindung betrifft eine Magnetresonanzvorrichtung mit Schalteinheit und ein Verfahren zum Betrieb einer solchen.

Es wird eine Magnetresonanzvorrichtung vorgeschlagen, die eine Leistungsverstärkungseinheit umfasst, die ausgebildet ist, mittels der Magnetresonanzvorrichtung auszusendende Sendesignale bereitzustellen, und eine Schalteinheit umfasst. Die Schalteinheit umfasst eine Aktoreinheit, ein erstes Steckverbindungsteil, das mit der Leistungsverstärkungseinheit verbunden ist, und mehrere zweite Steckverbindungsteile. Dabei ist die Aktoreinheit ausgebildet, das erste Steckverbindungsteil relativ zu den mehreren zweiten Steckverbindungsteilen so zu bewegen, dass eine elektrische Verbindung zwischen dem ersten Steckverbindungsteil und einem vorbestimmten Steckverbindungsteil der mehreren zweiten Steckverbindungsteile hergestellt wird.

## Beschreibung

Die Erfindung betrifft eine Magnetresonanzvorrichtung mit Schalteinheit und ein Verfahren zum Betrieb einer solchen.

In der Medizintechnik zeichnet sich die Bildgebung mittels Magnetresonanz (MR), auch Magnetresonanztomographie (MRT, engl. Magnetic Resonance Imaging, MRI) genannt, durch hohe Weichteilkontraste aus. Hierbei wird typischerweise ein menschlicher oder tierischer Patient in einem Hauptmagnetfeld einer Magnetresonanzvorrichtung positioniert. Während einer Magnetresonanzmessung werden üblicherweise mit Hilfe einer Hochfrequenzantenneneinheit einer Magnetresonanzvorrichtung hochfrequente (HF) Pulse in den Patienten eingestrahlt. Durch die erzeugten HF-Pulse werden im Patienten Kernspins angeregt, wodurch ortskodierte Magnetresonanzsignale ausgelöst werden. Die Magnetresonanzsignale werden von der Magnetresonanzvorrichtung empfangen und zur Rekonstruktion von Magnetresonanzabbildungen verwendet.

Zur Signalanregung bei Magnetresonanzvorrichtung mit ultrahohem Hauptmagnetfeld (UHF), z.B. 7 Tesla oder mehr, werden üblicherweise Lokalspulen, insbesondere körperregionsspezifische Sende-Empfangs-Spulen, verwendet. Im Gegensatz zu den niedrigeren Feldstärken, z.B. 3 Tesla oder weniger, hat sich der Einsatz einer Ganzkörpersendespule (engl. body coil) nicht etablieren können, insbesondere wegen ihrer typischerweise schlechten Sende- und SAR-Effizienz. In der Folge ist es bei einer MR-Messung mit einer UHF-Magnetresonanzvorrichtung notwendig, für jede Messregion einen Spulenwechsel durchzuführen.

Um eine Lokalspule anzuschließen, weist die Magnetresonanzvorrichtung am Patiententisch oftmals eine oder mehrere Lokalspulenanschlusseinheiten auf, beispielsweise in Form einer oder mehrerer Steckbuchsen. Je nachdem, welche Region gemessen werden soll, wird eine entsprechende Lokalspule an einer der mehreren Lokalspulenanschlusseinheiten angeschlossen. Jede der mehreren Lokalspulenanschlusseinheiten verfügt über eine Anzahl an Sende- bzw. Empfangskanäle. Um dem Arbeitsablauf und Sicherheitsanforderungen zu genügen, wird die Kabellänge einer steckbaren Lokalspule üblicherweise so kurz wie möglich gewählt. Daher wird die für eine bestimmte Messung geeignete Lokalspule mit einer nahegelegenen Lokalspulenanschlusseinheit verbunden. Jedoch ist die Gesamtzahl der Sende- bzw. Empfangskanäle der Magnetresonanzvorrichtung begrenzt, so dass auch die Anzahl der Lokalspulenanschlusseinheiten begrenzt und ihre räumliche Verteilung über den Patiententisch eingeschränkt ist.

Als Aufgabe der vorliegenden Erfindung kann angesehen werden, den Arbeitsablauf bei einer Messung unter Verwendung von Lokalspulen zu verbessern. Insbesondere kann auf Aufgabe angesehen werden, auf kosteneffiziente Weise mehrere Lokalspulenanschlusseinheiten zum Anschließen von Lokalspulen, insbesondere Sende-Empfangs-Spulen, bereitzustellen.

Die Aufgabe wird durch die Merkmale der unabhängigen Ansprüche gelöst. Vorteilhafte Ausgestaltungen sind in den abhängigen Ansprüchen beschrieben. Unabhängig vom grammatikalischen Geschlecht eines bestimmten Begriffes sind Personen mit männlicher, weiblicher oder anderer Geschlechteridentität mit umfasst.

Es wird eine Magnetresonanzvorrichtung vorgeschlagen, die eine Leistungsverstärkungseinheit umfasst, die ausgebildet ist, mittels der Magnetresonanzvorrichtung auszusendende Sendesignale bereitzustellen, und eine Schalteinheit umfasst. Die auszusendende Sendesignale sind beispielsweise zur Ausführung einer parallelen Sendetechnik (pTx) geeignet. Die Schalteinheit umfasst eine Aktoreinheit, ein erstes Steckverbindungsteil, das mit der Leistungsverstärkungseinheit verbunden ist, und mehrere zweite Steckverbindungsteile. Dabei ist die Aktoreinheit ausgebildet, das erste Steckverbindungsteil relativ zu den mehreren zweiten Steckverbindungsteilen so zu bewegen, insbesondere zu positionieren, dass eine elektrische Verbindung, insbesondere Steckverbindung, zwischen dem ersten Steckverbindungsteil und einem vorbestimmten Steckverbindungsteil der mehreren zweiten Steckverbindungsteile hergestellt wird. Insbesondere ist die Aktoreinheit ausgebildet, das erste Steckverbindungsteil zwischen den mehreren zweiten Steckverbindungsteilen umzustecken.

Vorteilhafterweise kann mit Hilfe der Schalteinheit eine bestimmte Anzahl an zur Verfügung stehenden Sendekanäle auf jeweils auf eine Lokalspulenanschlusseinheit geschalten werden. Insbesondere umfasst die Magnetresonanzvorrichtung mehrere Lokalspulenanschlusseinheiten, insbesondere Steckplätze, die jeweils ausgebildet sind, eine Lokalspule an die Magnetresonanzvorrichtung anzuschließen, wobei jeder der mehreren zweiten Steckverbindungsteile jeweils mit einer der mehreren Lokalspulenanschlusseinheiten elektrisch verbunden ist. Die Erfindung vereinfacht vorteilhafterweise eine Aufteilung einer HF-Sendeleistung auf mehrere Lokalspulenanschlusseinheiten bei geringem Aufwand für zusätzliche HF-Leitungen und/oder Umschaltkontakte.

Beispielsweise weist die Magnetresonanzvorrichtung, insbesondere die Leistungsverstärkungseinheit, N (N ≥ 1) Sendekanäle auf, die mit dem ersten Steckverbindungsteil verbunden sind; die Schalteinheit weist beispielsweise M (M > 1) zweite Steckverbindungsteile sowie M damit verbundene Lokalspulenanschlusseinheiten auf. Vorteilhafterweise kann dann jeder der M Lokalspulenanschlusseinheiten mit den N Sendekanälen über die Schalteinheit verbunden werden. Ohne Schalteinheit wären dazu üblicherweise NxM Sendekanäle erforderlich satt nur N Sendekanäle, d.h. vorteilhafterweise kann die Anzahl der Sendekanäle der Leistungsverstärkungseinheit (und die damit verbundene Hardware wie z.B. Verstärker etc.) reduziert werden.

Durch den Einsatz von Steckverbindungsteilen bzw. einer Steckverbindung kann Verbindung bereitgestellt werden, die eine hohe Störunempfindlichkeit, eine hohe Schaltleistung (üblicherweise erforderlicher Leistungsbereich ca. 8-32 kW Peak) und/oder nur geringe Verluste ermöglicht. Solche Steckverbindungen sind etwaigen Halbleiterschaltern somit deutlich überlegen. Ferner können vorteilhafterweise etwaige Reflexionen an offenen Enden vermieden werden. Vorteilhafterweise ist eine solch vorgeschlagene Steckverbindung magnetfeldgeeignet bzw. MR-kompatibel. Zudem kann die vorgeschlagene Schalteinheit vergleichsweise kostengünstig umgesetzt werden.

Das vorbestimmte zweite Steckverbindungsteil kann beispielsweise durch eine manuelle Auswahl einer mit dem zweiten Steckverbindungsteil verbundenen Lokalspulenanschlusseinheit durch das Bedienpersonal der Magnetresonanzvorrichtung vorbestimmt sein. Es ist auch denkbar, dass eine solche Auswahl automatisierst in Abhängigkeit einer durchzuführenden Magnetresonanzuntersuchung erfolgt. Soll beispielsweise eine Kopfuntersuchung durchgeführt werden, kann automatisch eine Verbindung mit dem zweiten Steckverbindungsteil hergestellt werden, das mit der Lokalspulenanschlusseinheit verbunden ist, die sich um Anschluss einer Kopfspule überhaupt eignet oder am besten eignet.

Vorteilhafterweise ist das erste Steckverbindungsteil zu jedem der mehreren zweiten Steckverbindungsteile mechanisch und/oder elektrisch korrespondierend. Insbesondere bildet das erste Steckverbindungsteil ein Gegenstück zu jedem der mehreren zweiten Steckverbindungsteile. Beispielsweise sind das erste Steckverbindungsteil ein Stecker und jedes der mehreren zweiten Steckverbindungsteile eine korrespondierende Steckdose.

Beispielsweise sind das erste Steckverbindungsteil ein Stecker und die mehreren zweiten Steckverbindungsteile zum Stecker korrespondierende Buchsen. Beispielsweise sind das erste Steckverbindungsteil eine Buchse und die mehreren zweiten Steckverbindungsteile zur Buchse korrespondierende Stecker.

Vorteilhafterweise bilden des erste Steckverbindungsteil und das damit verbundene zweite Steckverbindungsteil ein System zur Übertragung von Informationen und/oder elektrischer Energie. Vorteilhafterweise können über elektrische Verbindung die auszusendenden Sendesignale übertragen werden.

Vorteilhafterweise ist jedes der mehreren zweiten Steckverbindungsteile ausgebildet, mit dem ersten Steckverbindungsteil eine elektrische Verbindung einzugehen, so dass die Sendesignale über diese elektrische Verbindung übertragen werden können.

Insbesondere weist das erste Steckverbindungsteil eine erste, insbesondere geometrische, Form auf, und jedes der mehreren zweiten Steckverbindungsteile weist eine zweite, insbesondere geometrische, Form auf, wobei die erste Form und die zweite Form so aufeinander abgestimmt sind, dass sie bei Herstellung der elektrischen Verbindung mechanisch geführt werden und/oder ineinandergreifen.

Vorzugsweise weisen das erste Steckverbindungsteil und/oder die mehreren zweiten Steckverbindungsteile ein Gehäuse auf. Die Form des Steckverbindungsteils kann insbesondere durch die Form des Gehäuses des Steckverbindungsteils bedingt sein.

Vorzugsweise weisen das erste Steckverbindungsteils und/oder die mehreren zweiten Steckverbindungsteile zueinander korrespondierende elektrische Kontaktelemente, beispielsweise elektrische Kontaktstifte und/oder Kontaktöffnungen, auf. Vorzugsweise sind die Kontaktelemente ausgebildet (im verbundenen Zustand) einen leistungsfesten Kontakt zu bilden. Vorzugsweise ist ein einzelner Kontakt ausgelegt, eine Spitzenleistung von mehr als 0,5 kW, insbesondere mehr als 1 kW, insbesondere mehr als 1,5 kW, zu übertragen. Vorzugsweise ist ein einzelner Kontakt ausgelegt, eine mittlere Leistung von mehr als 50 W, insbesondere mehr als 100 W, insbesondere mehr als 150 W, zu übertragen. Vorzugsweise ist die Schalteinheit Kontakt ausgelegt, eine Summen-Spitzenleistung von mehr als 8 kW, insbesondere mehr als 12 kW, insbesondere mehr als 16 kW, zu übertragen. Vorzugsweise ist ein einzelner Kontakt ausgelegt, eine mittlere Leistung von mehr als 0,8 kW, insbesondere mehr als 1,2 kW, insbesondere mehr als 1,6 kW, zu übertragen.

Vorzugsweise weisen das erste Steckverbindungsteil und/oder die mehreren zweiten Steckverbindungsteile zumindest einen elektrischen Kontaktstift und/oder zumindest eine dazu korrespondierende Kontaktöffnung auf. Die Form der Steckverbindungsteile können insbesondere durch elektrische Kontaktstifte des Steckverbindungsteils und/oder deren Aufnahmen am korrespondierenden Steckverbindungsteil bedingt sein. Die elektrischen Kontaktstifte sind üblicherweise nach außen weisend (männlicher Teil einer Steckverbindung). Die Aufnahmen sind üblicherweise nach innen weisende Kontaktöffnungen (weiblicher Teil einer Steckverbindung). Ein Steckverbindungsteil kann gleichzeitig sowohl Kontaktstifte als auch Kontaktöffnungen aufweisen, d.h. Steckelemente beiderlei Geschlechts.

Vorzugsweise umfassen das erste Steckverbindungsteil und/oder die mehreren zweiten Steckverbindungsteile zumindest ein koaxiales elektrisches Kontaktelement.

Beispielsweise umfassen das erste Steckverbindungsteil und/oder die mehreren zweiten Steckerbindungsteile zumindest einen koaxialen Kontaktstift. Eine koaxiale Verbindung ermöglicht vorteilhafterweise eine besonders rauscharme Signalübertragung.

Vorzugsweise umfassen das erste Steckverbindungsteil und/oder die mehreren zweiten Steckverbindungsteile zumindest ein mechanisches Führungselement.

Beispielsweise weisen das erste Steckverbindungsteils und/oder die mehreren zweiten Steckverbindungsteile zumindest einen mechanischen Führungsstift und/oder zumindest eine dazu korrespondierende Führungsöffnung auf. Vorteilhafterweise ist der zumindest eine mechanische Führungsstift massiv ausgeführt.

Beispielsweise umfasst das erste Steckverbindungsteil einen mechanischen Führungsstift und die mehreren zweiten Steckverbindungsteile jeweils eine zum mechanischen Führungsstift korrespondierende Führungsöffnung.

Vorteilhafterweise ermöglicht der zumindest eine mechanische Führungsstift zusammen mit der zumindest einen dazu korrespondierenden Führungsöffnung einen präzisen und/oder zuverlässigen Steckvorgang des erste Steckverbindungsteils mit dem jeweiligen zweiten Steckverbindungsteil.

Vorzugsweise umfasst die Aktoreinheit zumindest einen Aktor. Der Aktor ist insbesondere ein Bauelement, das ein, insbesondere elektrisches, Steuersignal, in mechanische Bewegung umsetzt. Das Steuersignal kann beispielsweise von einer Systemsteuereinheit der Magnetresonanzvorrichtung an die Aktoreinheit übermittelt werden. Vorzugsweise umfasst die Aktoreinheit eine Aktorsteuereinheit, die ausgebildet ist, Steuersignale der Systemsteuereinheit zu empfangen.

Vorzugsweise ist die Systemsteuereinheit ausgebildet, die Steuersignale in Abhängigkeit einer durchzuführenden Magnetresonanzsequenz, insbesondere einer oder mehrerer dafür ausgewählten Lokalspulen, an die Aktorsteuereinheit zu senden. Wird gemäß der Magnetresonanzsequenz beispielsweise eine Messung des Kopfes des Patienten durchgeführt, wird mittels der Aktoreinheit vorteilhafterweise eine Verbindung des ersten Steckverbindungsteils mit dem zweiten Steckverbindungsteil hergestellt, das mit der Lokalspulenanschlusseinheit verbunden ist, an welche zum Anschluss einer Kopfspule geeignet ist.

Beispielsweise umfasst die Aktoreinheit, insbesondere der zumindest eine Aktor, zumindest einen pneumatischen Antrieb. Dabei werden vorzugsweise mittels eines Gases, beispielsweise einer Druckluft, Kräfte zur Bewegung des erste Steckverbindungsteils übertragen.

Beispielsweise umfasst die Aktoreinheit, insbesondere der zumindest eine Aktor, zumindest einen hydraulischen Antrieb. Dabei werden vorzugsweise mittels einer Flüssigkeit, beispielsweise eines (zu etwaigen Normen kompatibles) Öls, Kräfte zur Bewegung des erste Steckverbindungsteils übertragen.

Der zumindest eine pneumatische Antrieb und/oder der zumindest eine hydraulischen Antrieb sind vorzugsweise ausgebildet, Druckenergie in Bewegungsenergie umzusetzen, um das erste Steckverbindungsteil zu bewegen.

Beispielsweise umfasst die Aktoreinheit, insbesondere der zumindest eine Aktor, zumindest einen Elektromotor. Der zumindest eine Elektromotor ist vorzugsweise ein elektromechanischer Wandler, der elektrische Leistung in mechanische Leistung umwandelt, um das erste Steckverbindungsteil zu bewegen.

Vorzugsweise ist der zumindest eine Elektromotor geschirmt. Vorteilhafterweise werden durch die Schirmung Störungen reduziert.

Vorzugsweise umfasst die Magnetresonanzvorrichtung einen Magneten zur Erzeugung eines Hauptmagnetfeldes, wobei der zumindest eine Elektromotor einen Rotor umfasst, der mit dem Hauptmagnetfeld der Magnetresonanzvorrichtung als Permanentmagnetfeld des Elektromotors zusammenwirkt. Vorteilhafterweise kann also das ohnehin vorhandene Hauptmagnetfeld zum Betrieb des Elektromotors verwendet werden.

Vorteilhafterweise sind die vorangehend vorgeschlagenen Aktoren geeignet, einen Umschaltvorgang auch in einem starken Magnetfeld durchzuführen.

Vorzugsweise umfasst die Aktoreinheit einen Rollenauszug. Das erste Steckverbindungsteil kann insbesondere an einer Laufschiene angeordnet sein. Dabei kommt beispielsweise eine Sendeleitung zum Einsatz, die einen Durchmesser von ca. 5 mm und einen Biegekreisdurchmesser ca. 8 cm aufweist. Vorstellbar ist beispielsweise ein Wickeldorn mit ca. 10 cm Durchmesser und Federrückstellkraft. Hier würden insbesondere zwei Wicklungen genügen, um ca. 50 cm Fahrweg für das Steckverbindungsteil bereitzustellen.

Vorzugsweise ist die Aktoreinheit ausgebildet, das erste Steckverbindungsteil in zumindest zwei Raumrichtungen zu bewegen (Bewegungsrichtungen). Beispielsweise sind die mehreren zweiten Steckverbindungsteile entlang einer Gerade angeordnet, wobei diese Gerade eine erste der Bewegungsrichtungen der Aktoreinheit entspricht.

Vorzugsweise sind die mehreren zweiten Steckverbindungsteile in einer Ebene angeordnet, wobei die Aktoreinheit ausgebildet ist, das erste Steckverbindungsteil parallel zu der Ebene zu verfahren, um das erste Steckverbindungsteil in die Nähe eines der mehreren zweiten Steckverbindungsteile und/oder in Deckung mit einem der mehreren zweiten Steckverbindungsteile zu bringen.

Eine zweite Bewegungsrichtung der Aktoreinheit ist beispielsweise eine Richtung senkrecht zu ersten Bewegungsrichtung, um durch Bewegung in dieser Richtung das erste Steckverbindungsteil mit einem der mehreren zweiten Steckverbindungsteile in Verbindung zu bringen und die Verbindung durch Bewegung in entgegengesetzter Richtung auch wieder zu lösen.

Vorzugsweise sind mehreren zweiten Steckverbindungsteile in einer Ebene angeordnet, wobei die Aktoreinheit ausgebildet ist, das erste Steckverbindungsteil senkrecht zu der Ebene zu verschieben, um das erste Steckverbindungsteil mit einem der mehreren zweiten Steckverbindungsteile in Verbindung zu bringen und die Verbindung durch Bewegung in entgegengesetzter Richtung auch wieder zu lösen.

Vorteilhafterweise ist die Schalteinheit im selben Raum angeordnet, in dem sich auch die (restliche) Magnetresonanzvorrichtung, insbesondere deren Magneteinheit, befindet. Vorzugsweise umfasst die Magnetresonanzvorrichtung einen Patiententisch und eine Magneteinheit zur Erzeugung eines Hauptmagnetfeldes der Magnetresonanzvorrichtung, wobei die Schalteinheit im Bereich des Patiententisches und/oder in der Nähe der Magneteinheit angeordnet ist.

Durch die Anordnung der Schalteinheit an der Magnetresonanzvorrichtung können vorteilhafterweise kurze Signalleitungen ermöglicht werden. Eine mögliche Anordnung der Schalteinheit in einem dem Raum, in dem die Magnetresonanzvorrichtung steht, benachbarten Raum, würde dagegen relativ lange Signalleitungen erfordern. Damit können auch sonst vorhandene Leitungsverluste minimiert werden, so dass die Spannungsfestigkeitsanforderungen an die elektrischen Verbindungen deutlich reduziert werden können.

Vorzugsweise weist die Magnetresonanzvorrichtung ein Hauptmagnetmagnet eine Stärke von mindestens 5 Tesla auf. Bei solchen Magnetresonanzvorrichtungen können Lokalspule, insbesondere HF-Sende-Spulen, mittels der Lokalspulenanschlusseinheiten besonders effektiv eingesetzt werden.

Vorzugsweise umfasst die Magnetresonanzvorrichtung zumindest eine Lokalspule, die ausbildet ist, hochfrequente Pulse gemäß der bereitgestellten Sendesignale in einen Untersuchungsbereich der Magnetresonanzvorrichtung zu auszusenden.

Ferner wird ein Verfahren zur Sicherstellung einer korrekten Verbindung vorgeschlagen. Dabei wird mit vorab beschriebenen einer Magnetresonanzvorrichtung zumindest ein hochfrequenter Puls ausgesendet wird und dessen Reflexion in Amplitude und/oder Phase ausgewertet. Vorteilhafterweise kann anhand der ausgewerteten Information festgestellt werden, ob die Schalteinheit eine Verbindung mit dem richtigen zweiten Steckverbindungsteil und der damit verbundenen Lokalspulenanschlusseinheit hergestellt hat.

Vorzugsweise wird der zumindest eine hochfrequente Puls zu Beginn einer Durchführung einer Magnetresonanzsequenz ausgesendet. Vorteilhafterweise kann dadurch ein möglicher Fehler frühzeitig festgestellt werden.

Vorzugsweise weist der zumindest eine hochfrequente Puls eine geringe Amplitude auf. Vorteilhafterweise kann die Überprüfung dadurch besonders sicher durchgeführt werden.

Die Merkmale, Vorteile oder alternative Ausführungsformen der vorgeschlagenen Magnetresonanzvorrichtung, welche vorab im Detail ausgeführt sind, können auf das vorgeschlagene Verfahren zur Sicherstellung einer korrekten Verbindung übertragen werden und umgekehrt.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen in schematischer Darstellung:
- Fig. 1: eine Magnetresonanzvorrichtung,
- Fig. 2: eine Schalteinheit mit einer Reihe zweiter Steckverbindungsteile,
- Fig. 3: eine Schalteinheit mit zwei Reihen zweiter Steckverbindungsteile,
- Fig. 4: mögliche Anordnungen der Schalteinheit an der Magnetresonanzvorrichtung,
- Fig. 5: ein Verfahren zur Sicherstellung einer korrekten Verbindung.

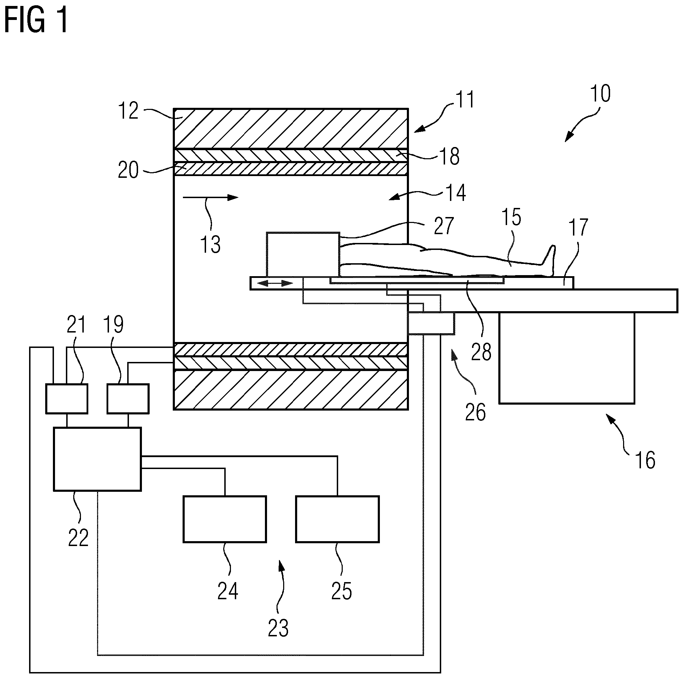

In Fig. 1 ist eine Magnetresonanzvorrichtung 10 schematisch dargestellt. Die Magnetresonanzvorrichtung 10 umfasst eine Magneteinheit 11, die einen Hauptmagneten 12 zu einem Erzeugen eines starken und insbesondere zeitlich konstanten Hauptmagnetfelds 13 aufweist. Das Hauptmagnetfeld 13 ist insbesondere eine Ultrahochmagnetfeld. Zudem umfasst die Magnetresonanzvorrichtung 10 einen Patientenaufnahmebereich 14 zu einer Aufnahme eines Patienten 15. Der Patientenaufnahmebereich 14 im vorliegenden Ausführungsbeispiel ist zylinderförmig ausgebildet und in einer Umfangsrichtung von der Magneteinheit 11 zylinderförmig umgeben. Der Patient 15 kann mittels einer Patientenlagerungsvorrichtung 16 der Magnetresonanzvorrichtung 10 in den Patientenaufnahmebereich 14 geschoben werden. Die Patientenlagerungsvorrichtung 16 weist hierzu einen innerhalb des Patientenaufnahmebereichs 14 bewegbar ausgestalteten Patiententisch 17 auf.

Die Magneteinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Magneteinheit 11 umfasst weiterhin eine Hochfrequenzantenneneinheit 20, welche im vorliegenden Ausführungsbeispiel als fest in die Magnetresonanzvorrichtung 10 integrierte Körperspule ausgebildet ist. Anders als hier dargestellt, weisen UHF-Magnetresonanzvorrichtungen üblicherweise jedoch keine fest in die Magnetresonanzvorrichtung 10 integrierte Körperspule auf; vielmehr werden körperregionsspezifische Lokalspulen, wie hier eine Kopfspule 27 oder eine Wirbelsäulenspule 28, verwendet, die ausgebildet sind, hochfrequente Magnetresonanzsequenzen in einen Untersuchungsraum einzustrahlen, der im Wesentlichen von einem Patientenaufnahmebereich 14 der Magnetresonanzvorrichtung 10 gebildet ist. Dadurch stellt sich in dem von dem Hauptmagneten 12 erzeugten Hauptmagnetfeld 13 eine Anregung von Atomkernen ein. Durch Relaxation der angeregten Atomkerne werden Magnetresonanzsignale erzeugt. Die Lokalspulen sind hier sowohl zum Senden von HF-Signalen aus auch zum Empfang der Magnetresonanzsignale ausgebildet.

Zu einer Steuerung des Hauptmagneten 12, der Gradientensteuereinheit 19 und zur Steuerung der Hochfrequenzantennensteuereinheit 21 weist die Magnetresonanzvorrichtung 10 eine Systemsteuereinheit 22 auf. Die Systemsteuereinheit 22 steuert zentral die Magnetresonanzvorrichtung 10, wie beispielsweise das Durchführen einer vorbestimmten bildgebenden Gradientenechosequenz. Zudem umfasst die Systemsteuereinheit 22 eine nicht näher dargestellte Auswerteeinheit zu einer Auswertung der Magnetresonanzsignale, die während der Magnetresonanzuntersuchung erfasst werden. Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzabbildungen können auf einer Anzeigeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

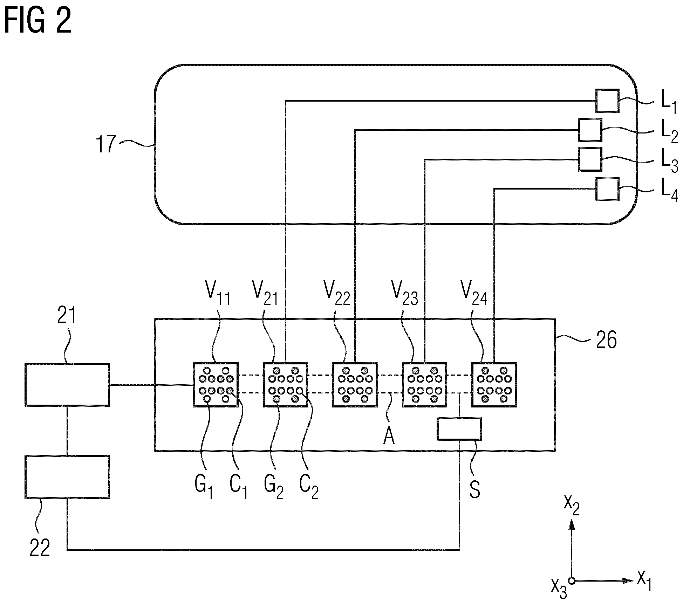

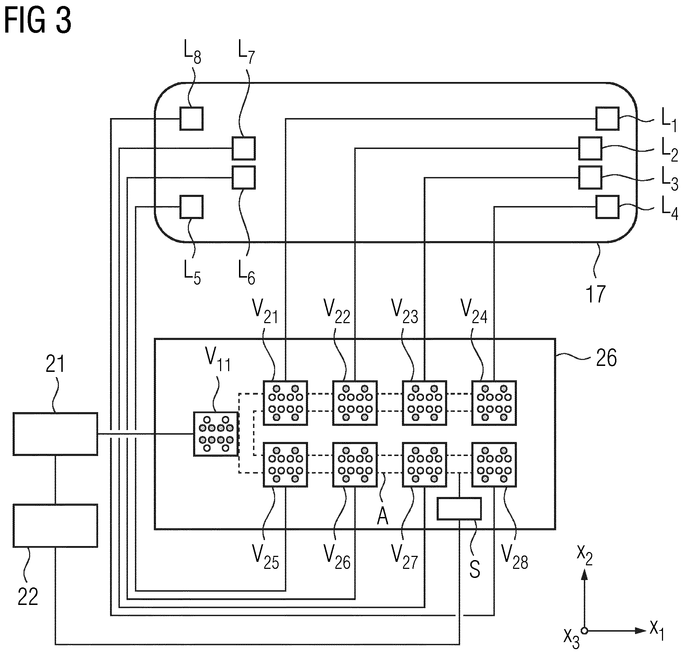

Zum Betrieb der Lokalspulen umfasst die Magnetresonanzvorrichtung 10 eine Hochfrequenzantennensteuereinheit 21, die eine Leistungsverstärkungseinheit mit mehreren Sendekanäle aufweist. Die Leistungsverstärkungseinheit ist ausgebildet ist, auszusendende Sendesignale bereitzustellen, um beispielsweise pTx-Pulse zu erzeugen. Um die zur Verfügung stehenden Sendekanäle zu schalten, umfasst die Magnetresonanzvorrichtung eine Schalteinheit 26, die in Fig. 2 und 3 näher beschrieben wird. Die Sendeleitungen von der Leistungsverstärkungseinheit enden an einem ersten Steckverbindungsteil der Schalteinheit (vgl. Fig. 2 und 3).

In Fig. 2 ist eine Schalteinheit dargestellt, die eine Aktoreinheit, ein erstes Steckverbindungsteil V₁₁, das mit der Leistungsverstärkungseinheit der Hochfrequenzantennensteuereinheit 21 verbunden ist, und vier zweite Steckverbindungsteile V₂₁, V₂₂, V₂₃, V₂₄ umfasst. Die Aktoreinheit A ist ausgebildet ist, das erste Steckverbindungsteil V₁₁ relativ zu den vier zweiten Steckverbindungsteile so zu positionieren, dass eine elektrische Verbindung zwischen dem ersten Steckverbindungsteil V₁₁ und einem der mehreren zweiten Steckverbindungsteile V₂₁, V₂₂, V₂₃, oder V₂₄ hergestellt wird, das insbesondere zuvor ausgewählt wurde.

Dazu ist die Aktoreinheit A in der Lage, das erste Steckverbindungsteil V₁₁ in der Richtung ±x₁ entlang der Steckverbindungsteile V₂₁, V₂₂, V₂₃, V₂₄, die entlang einer Gerade parallel zur Richtung x₁ angeordnet sind, zu bewegen. (Die Richtungen x₁, x₂, x₃ in Fig. 2 und 3 sind vorzugsweise zueinander orthogonal.) Durch eine Bewegung in dieser Richtung kann also das jeweils gewünschte zweite Steckverbindungsteile V₂₁, V₂₂, V₂₃ oder V₂₄ angefahren werden. (Beispielsweise steht das erste Steckverbindungsteil V₁₁ nach einer solchen Anfahrt über dem zweite Steckverbindungsteil V₂₃.)

Ferner ist die Aktoreinheit A in der Lage, das erste Steckverbindungsteil V₁₁ in der Richtung ±x₃ zu bewegen. Durch Bewegung in diese Richtung kann eine Verbindung zwischen dem erste Steckverbindungsteil V₁₁ und dem gewünschten zweite Steckverbindungsteile V₂₁, V₂₂, V₂₃ oder V₂₄ hergestellt oder auch wieder gelöst werden. (Beispielsweise wird das nach der Anfahrt über dem zweiten Steckverbindungsteil V₂₃ stehende Steckverbindungsteil V₁₁ nach unten in das Steckverbindungsteil V₂₃ bewegt, um eine elektrische Verbindung herzustellen.)

Um die Bewegungen zu steuern, umfasst die Schalteinheit 26 ferner eine Aktorsteuereinheit S, die von der Systemsteuereinheit 22 Steuersignale empfangen kann und diese in entspreche Bewegungen des ersten Steckverbindungsteil V₁₁ mittels der Aktoreinheit A umsetzen kann. Die Systemsteuereinheit 22 steuert zudem die Hochfrequenzantennensteuereinheit 21 mit ihrer Leistungsverstärkungseinheit, die im vorliegenden Fall acht Leistungsverstärker für jeweils einen Sendekanal umfasst. Dementsprechend weist das erste Steckverbindungsteil V₁₁ auch acht elektrische Kontaktelemente C₁ auf. Solche Kontaktelemente können beispielsweise elektrische Kontaktstifte sein, die in dazu korrespondierende Kontaktelemente C₂, insbesondere Kontaktöffnungen, bei Stecken des ersten Steckverbindungsteils V₁₁ in das gewünschte zweite Steckverbindungsteile V₂₁, V₂₂, V₂₃ oder V₂₄ eine elektrische Verbindung eingehen.

Um einen zuverlässige Verbindungsvorgang, insbesondere Steckvorgang, durchzuführen, können die Steckverbindungsteile V₁₁ sowie V₂₁, V₂₂, V₂₃ oder V₂₄ ineinander eingreifende Führungselemente G₁ bzw. G₂ aufweisen. Das können beispielsweise, insbesondere massive, Führungsstifte sein, die in eine gegenüberliegende Öffnung beim Verbindungsvorgang eintauchen.

Jedes der vier zweiten Steckverbindungsteile V₁₁ sowie V₂₁, V₂₂, V₂₃, V₂₄ ist wiederum mit einer entsprechenden Lokalspulenanschlusseinheit L₁, L₂, L₃ oder L₄ elektrisch verbunden. Im verbundenen Zustand können die von der Leistungsverstärkereinheit bereitgestellten acht Sendesignale über die miteinander verbundenen Steckverbindungsteile zu der jeweiligen Lokalspulenanschlusseinheit L₁, L₂, L₃ oder L₄ übertragen werden.

Die Lokalspulenanschlusseinheit L₁, L₂, L₃, L₄ sind am Patiententisch 17 angeordnet. Soll beispielsweise an die Lokalspulenanschlusseinheit L₄ eine Lokalspule angeschlossen und betrieben werden, so wird das erste Steckverbindungsteil V₁₁ in das zweite Steckverbindungsteil V₂₄ mittels der Aktoreinheit A gesteckt, um die Sendekanäle der Leistungsverstärkereinheit mit der Lokalspulenanschlusseinheit L₄ elektrisch zu verbinden.

In Fig. 3 ist eine Schalteinheit 26 mit acht zweiten Steckverbindungsteilen V₂₁, V₂₂, V₂₃, V₂₄, V₂₅, V₂₆, V₂₇, V₂₈ dargestellt, die in zwei Reihen angeordnet sind, die in der Richtung x₂ zueinander versetzt sind. Die acht zweiten Steckverbindungsteilen V₂₁, V₂₂, V₂₃, V₂₄, V₂₅, V₂₆, V₂₇, V₂₈ sind jeweils mit einer Lokalspulenanschlusseinheit L₁, L₂, L₃, L₄, L₅, L₆, L₇, L₈ elektrisch verbunden. Vorteilhafterweise ist in diesem Fall die Aktoreinheit ausgebildet, das erste Steckverbindungsteil V₁₁ auch entlang der Richtung x₂ zu bewegen, um eine Verbindung mit einem der zweiten Steckverbindungsteilen V₂₁, V₂₂, V₂₃, V₂₄, V₂₅, V₂₆, V₂₇ oder V₂₈ herstellen zu können.

Die Lokalspulenanschlusseinheit L₁, L₂, L₃, L₄, L₅, L₆, L₇, L₈ sind hier an beiden Enden des Patiententisches 17 verteilt. Vorteilhafterweise kann so leichter auch bei kurzer Kabellänge einer Lokalspule diese an der richtigen Stelle zur Messung positioniert werden. Somit ist auch ein Spulenwechsel mit Umsteckung der Lokalspulen besser möglich.

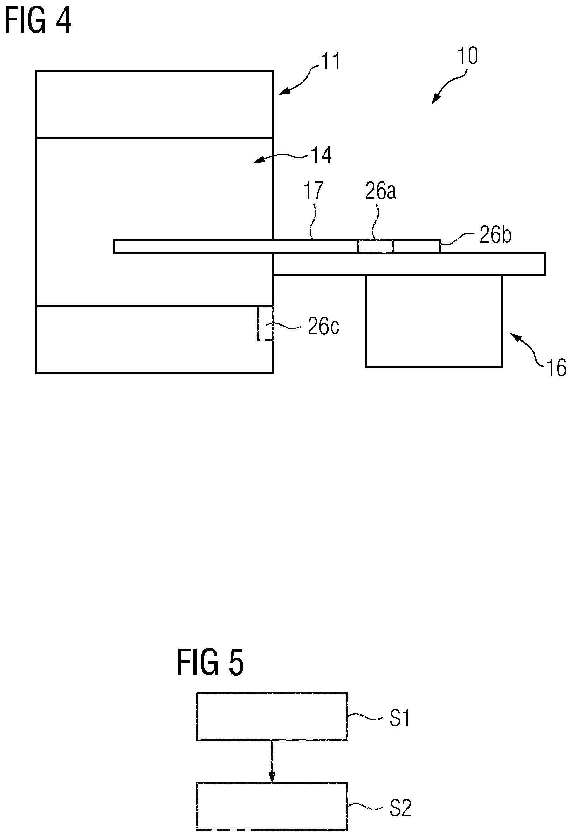

In Fig. 4 werden Positionen gezeigt, die sich besonders zur Anordnung der Schalteinheit eignen. So kann diese beispielsweise am Ende des Patiententisches 17 angeordnet sein, 26a, oder hinter dem Patiententisch, 26b, oder an der Vorderseite der Magneteinheit 11 hinter deren Abdeckung (insbesondere dann, wenn die Sendeleitungen von dort in die Liege gehen).

In Fig. 5 ist ein Verfahren zur Sicherstellung einer korrekten Verbindung dargestellt. In S1 stellt die Leistungsverstärkungseinheit ein auszusendendes Sendesignal zur Erzeugung eines hochfrequenten Pulses bereit.

In S2 wird ein durch eine Empfangseinheit der Magnetresonanzvorrichtung (beispielsweise der Lokalspule, wenn sie eine Sende-Empfangs-Spule ist) gemessenes Signal in Amplitude und/oder Phase ausgewertet. Ist das gemessene Signal beispielsweise zu gering, kann daraus geschlossen werden, dass keine korrekte Verbindung zwischen Leistungsverstärkungseinheit und Sendeeinheit (beispielsweise der Lokalspule, wenn sie eine Sende-Empfangs-Spule ist) gegeben war. Dies kann insbesondere dadurch bedingt sein, dass Schalteinheit keine korrekte Verbindung hergestellt hat.

S1 und S2 erfolgen vorzugsweise zu Beginn einer Durchführung einer Magnetresonanzsequenz. Das auszusendende Sendesignal weist vorzugsweise eine geringe Amplitude auf.

Es wird abschließend noch einmal darauf hingewiesen, dass es sich bei der vorhergehend detailliert beschriebenen Magnetresonanzvorrichtung sowie bei dem Verfahren lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließt der Begriff "Einheit" nicht aus, dass die betreffenden Komponenten aus mehreren zusammenwirkenden TeilKomponenten bestehen, die gegebenenfalls auch räumlich verteilt sein können.

## Patentansprüche

1. Magnetresonanzvorrichtung, die umfasst:
eine Leistungsverstärkungseinheit, die ausgebildet ist, auszusendende Sendesignale bereitzustellen,
eine Schalteinheit, wobei die Schalteinheit eine Aktoreinheit, ein erstes Steckverbindungsteil, das mit der Leistungsverstärkungseinheit verbunden ist, und mehrere zweite Steckverbindungsteile umfasst,
wobei die Aktoreinheit ausgebildet ist, das erste Steckverbindungsteil relativ zu den mehreren zweiten Steckverbindungsteilen so zu bewegen, dass eine elektrische Verbindung zwischen dem ersten Steckverbindungsteil und einem der mehreren zweiten Steckverbindungsteile hergestellt wird.

2. Magnetresonanzvorrichtung nach Anspruch 1,
wobei die Aktoreinheit zumindest einen pneumatischen Antrieb und/oder zumindest einen hydraulischen Antrieb umfasst.

3. Magnetresonanzvorrichtung nach einen der Ansprüche 1 oder 2,
wobei die Aktoreinheit zumindest einen, insbesondere geschirmten, Elektromotor umfasst.

4. Magnetresonanzvorrichtung nach Anspruch 3,
wobei die Magnetresonanzvorrichtung eine Magneteinheit zur Erzeugung eines Hauptmagnetfeldes umfasst,
wobei der zumindest eine Elektromotor einen Rotor umfasst, der mit dem Hauptmagnetfeld der Magnetresonanzvorrichtung als Permanentmagnetfeld des Elektromotors zusammenwirkt.

5. Magnetresonanzvorrichtung nach einem der vorangehenden Ansprüche,
wobei mehreren zweiten Steckverbindungsteile in einer Ebene angeordnet sind,
wobei die Aktoreinheit ausgebildet ist, das erste Steckverbindungsteil parallel zu der Ebene zu verfahren, um das erste Steckverbindungsteil in die Nähe eines der mehreren zweiten Steckverbindungsteile, insbesondere deckungsgleich zu einem der mehreren zweiten Steckverbindungsteile, zu bringen.

6. Magnetresonanzvorrichtung nach einem der vorangehenden Ansprüche,
wobei mehreren zweiten Steckverbindungsteile in einer Ebene angeordnet sind,
wobei die Aktoreinheit ausgebildet ist, das erste Steckverbindungsteil senkrecht zu der Ebene zu verfahren, um das erste Steckverbindungsteil mit einem der mehreren zweiten Steckverbindungsteile lösbar in Verbindung zu bringen.

7. Magnetresonanzvorrichtung nach einem der vorangehenden Ansprüche,
wobei die Magnetresonanzvorrichtung einen Patiententisch umfasst,
wobei die Magnetresonanzvorrichtung eine Magneteinheit zur Erzeugung eines Hauptmagnetfeldes der Magnetresonanzvorrichtung umfasst,
wobei die Schalteinheit im Bereich des Patiententisches und/oder in der Nähe der Magneteinheit angeordnet ist.

8. Magnetresonanzvorrichtung nach einem der vorangehenden Ansprüche,
wobei das erste Steckverbindungsteil und/oder die mehreren zweiten Steckverbindungsteile zumindest ein koaxiales elektrisches Kontaktelement, insbesondere zumindest einen koaxialen Kontaktstift, umfasst.

9. Magnetresonanzvorrichtung nach einem der vorangehenden Ansprüche,
wobei das erste Steckverbindungsteil und/oder die mehreren zweiten Steckverbindungsteile zumindest ein mechanisches Führungselement, insbesondere zumindest einen mechanischen Führungsstift, umfasst.

10. Magnetresonanzvorrichtung nach einem der vorangehenden Ansprüche,
wobei die Magnetresonanzvorrichtung eine Systemsteuereinheit umfasst,
wobei die Aktoreinheit eine Aktorsteuereinheit umfasst, die ausgebildet ist, Steuersignale der Systemsteuereinheit zu empfangen.

11. Magnetresonanzvorrichtung nach Anspruch 10,
wobei die Systemsteuereinheit ausgebildet ist, die Steuersignale in Abhängigkeit einer durchzuführenden Magnetresonanzsequenz an die Aktorsteuereinheit zu senden.

12. Magnetresonanzvorrichtung nach einem der vorangehenden Ansprüche,
wobei die Magnetresonanzvorrichtung mehrere Lokalspulenanschlusseinheiten umfasst, die jeweils ausgebildet sind, eine Lokalspule an die Magnetresonanzvorrichtung anzuschließen, wobei jeder der mehreren zweiten Steckverbindungsteile jeweils mit einer der mehreren Lokalspulenanschlusseinheiten elektrisch verbunden ist.

13. Magnetresonanzvorrichtung nach einem der vorangehenden Ansprüche,
wobei die Magnetresonanzvorrichtung zumindest eine Lokalspule umfasst, die ausbildet ist, hochfrequente Pulse gemäß der bereitgestellten Sendesignale in einen Untersuchungsbereich der Magnetresonanzvorrichtung zu auszusenden.

14. Verfahren zur Sicherstellung einer korrekten Verbindung, wobei mit einer Magnetresonanzvorrichtung nach einem der vorangehenden Ansprüche, insbesondere zu Beginn einer Durchführung einer Magnetresonanzsequenz, ein auszusendendes Sendesignal zur Erzeugung zumindest ein hochfrequenter Puls, insbesondere geringer Amplitude, bereitgestellt wird und ein durch eine Empfangseinheit der Magnetresonanzvorrichtung gemessenes Signal ausgewertet wird.

15. Verfahren nach Anspruch 14,
wobei das gemessene Signal hinsichtlich seiner Amplitude und/oder Phase ausgewertet wird.
